# FASCICULE DE BREVET EUROPEEN

(11) **EP 1 289 582 B1**
(45) Date de publication et mention de la délivrance du brevet: **07.09.2005**
(21) Numéro de dépôt: 01938358.7
(22) Date de dépôt: 29.05.2001
(51) Int. Cl.: A61M 5/30

(54) **SERINGUE SANS AIGUILLE AVEC MEMBRANE D'ISOLATION D'UN INJECTEUR MULTICONDUIT**
NADELLOSE SPRITZE MIT EINEM EINE ISOLATIONSMEMBRAN ENTHALTENDEN MEHRFACHKANALINJEKTOR
NEEDLELESS SYRINGE WITH MEMBRANE ISOLATING A MULTIPLE DUCT INJECTOR

(30) Priorité: 30.05.2000 FR 0006925
(43) Date de publication de la demande: 12.03.2003
(73) Titulaire: Crossject, 75181 Paris Cédex 04 (FR)
(72) Inventeur: ALEXANDRE, Patrick, F-70100 Gray (FR); BROUQUIERES, Bernard, F-83100 Toulon (FR); GAUTIER, Philippe, F-91220 Le Plessis Pate (FR); NAVELIER, Alain, F-83390 Pierrefeu du Var (FR)
(86) Numéro de dépôt international: PCT/FR2001/001645
(87) Numéro de publication internationale: WO 2001/091835

(56) Documents cités:
- WO-A-00/33899
- WO-A-96/15821
- FR-A- 2 629 348
- GB-A- 971 162
- US-A- 2 764 977
- US-A- 4 124 024
- US-A- 5 769 138

## Description

La présente invention est dans le domaine des seringues sans aiguille, prèremplies et jetables ; de telles seringues sont utilisées pour les injections intradermiques, sous-cutanées et intramusculaires, de principe actif liquide à usage thérapeutique en médecine humaine ou vétérinaire. Une seringue selon le préambule de la revendication 1 est connue du document GB-A- 971 162.

Un premier impératif pour des seringues préremplies est celui de la compatibilité à long terme, trois ans en général, entre le principe actif liquide et le réservoir qui le contient. Un autre impératif, lié au procédé de préremplissage, est d'avoir un réservoir transparent pour faire les contrôles réglementaires du remplissage correct du réservoir avant son montage dans la seringue. Ces impératifs conduisent à la réalisation d'un réservoir essentiellement transparent et en matériau compatible avec le principe actif pour la durée souhaitée ; c'est en général du verre à usage pharmaceutique : verre de type I ou II. Mais alors se pose le problème de la résistance mécanique de ce réservoir à la pression élevée de fonctionnement nécessaire pour réaliser l'injection sans aiguille.

Le brevet EP 792 174 décrit une seringue sans aiguille dont l'ampoule contenant le principe actif est en verre, cette ampoule est partiellement cylindrique, sa partie aval est en forme d'ogive, avec à son extrémité, un seul conduit d'injection, très court : en fait un orifice d'injection. Cette ampoule est frettée, sur sa partie cylindrique, dans une pièce de compression. Ce dispositif résout d'une part les problèmes de compatibilité à long terme entre le principe actif et le réservoir et d'autre part les problèmes de la résistance du réservoir à la pression de fonctionnement. Mais cette disposition n'est pas transposable à une seringue qui aurait plusieurs conduits d'injection et plus particulièrement des conduits longs par rapport à leur diamètre pour pouvoir piloter la distance de cohérence des jets et la profondeur de pénétration des jets dans la peau du sujet traité. Pour réaliser plusieurs conduits longs dans un réservoir résistant aux fortes pressions de fonctionnement des seringues sans aiguilles, on est amené à modifier l'organisation du réservoir avec une partie essentiellement tubulaire et éventuellement transparente et un injecteur résistant à la pression, mais pas forcément dans un matériau compatible pour une longue durée avec le principe actif et donc un moyen supplémentaire pour rétablir cette compatibilité.

La présente invention concerne une seringue sans aiguille pour l'injection d'un principe actif liquide contenu dans un réservoir cylindrique, fermé à sa partie amont par un obturateur déplaçable par un moyen moteur, et fermé à sa partie aval par un injecteur comprenant au moins un conduit d'injection et ce réservoir étant solidarisé au corps de la seringue qui est caractérisée en ce que une membrane, compatible avec le principe actif liquide, isole ce dernier de l'injecteur et que ladite membrane est traversable par le liquide lors de sa mise en pression pour l'injection.

Dans cette invention par principe actif liquide nous entendrons essentiellement un liquide plus ou moins visqueux, ou un mélange de liquides, ou un gel. Le principe actif pourra être un solide mis en solution dans un solvant approprié pour l'injection. Le principe actif pourra être un solide sous forme pulvérulente mis en suspension, plus ou moins concentrée, dans un liquide approprié. La granulométrie du principe actif solide doit être adaptée, ainsi que la forme du conduit pour éviter les bouchages.

Le moyen qui va agir sur l'obturateur déplaçable, peut être de type mécanique : détente d'un ressort comprimé ou du type pneumatique : détente de gaz comprimé, ou du type pyrotechnique : détente de gaz de combustion.

L'injecteur doit comporter plusieurs conduits d'injection dont la longueur et la forme permettent de contrôler la distance de cohérence du jet. L'injecteur doit résister à la pression de fonctionnement. Le matériau choisi pour sa réalisation doit satisfaire à ses deux exigences. Par contre il n'est pas nécessaire que ce matériau soit compatible, pour une longue durée, avec le principe actif : la membrane traversable qui isole l'injecteur du principe actif, va permettre cette compatibilité pour une longue durée. Le matériau de la membrane sera choisi parmi les matériaux de la pharmacopée connus pour être compatibles avec le principe actif, par exemple des élastomères du type de ceux utilisés pour réaliser les bouchons-pistons dans les seringues préremplies, ou des silicones, ou des polytétrafluoroéthylènes (PTFE). Le réservoir cylindrique qui va contenir le principe actif peut être en verre ou en tout autre matériau transparent, compatible avec le principe actif. Le réservoir sera lui-même capable de résister à la pression de fonctionnement ou sera disposé, d'une façon appropriée, dans un autre élément de la seringue qui lui permettra de résister à la pression de fonctionnement.

Avantageusement dans cette seringue la membrane traversable assure l'étanchéité entre le réservoir et l'injecteur. L'étanchéité est assurée soit par pincement de la membrane entre le réservoir et l'injecteur : la membrane agit alors comme un joint d'étanchéité, soit la membrane est engagée dans le réservoir : elle agit comme un bouchon.

Dans une première réalisation la membrane est assez mince pour qu'elle se perce, en regard des conduits d'injection, lorsque le liquide est mis sous forte pression pour faire l'injection.
Dans une deuxième réalisation la membrane comporte une zone de moindre épaisseur, en regard de chaque conduit d'injection ; chacune de ces zones se perce, comme précédemment, au moment de la mise en pression. Pour le bon fonctionnement de cette réalisation la membrane doit être bien positionnée, par des dispositifs appropriés, pour que chaque zone de moindre épaisseur soit en vis à vis d'un conduit d'injection. Une zone de moindre épaisseur est une cavité borgne faite dans la membrane, la base de cette cavité peut être dirigée soit vers le liquide soit vers la face amont de l'injecteur et plus précisément un conduit d'injection. Une des formes les plus simples est celle d'un cône ou d'un tronc de cône.
Dans une troisième réalisation la membrane comporte un préperçage en regard de chaque conduit d'injection. L'élasticité de l'élastomère de la membrane maintient fermé chacun des préperçages et éventuellement cette fermeture est étanche. Lorsque le liquide est mis en pression par le déclenchement du moyen moteur, chaque préperçage s'ouvre.

La membrane recouvre au moins deux conduits d'injection dont les extrémités amont sont situées sur un même cercle centré sur l'axe du réservoir cylindrique.

Avantageusement les zones de moindre épaisseur de la membrane se rejoignent et forment une gorge circulaire centrée sur l'axe du réservoir. Cette zone circulaire est en regard des extrémités amonts des conduits d'injections elles-mêmes réparties sur un même cercle. Comme précédemment, pour une zone de moindre épaisseur, l'ouverture de la gorge circulaire de la membrane est dirigée soit vers le liquide soit vers la face amont de l'injecteur.
Avantageusement encore la membrane comporte une fente circulaire centrée sur l'axe du réservoir, cette fente est en regard des extrémités amonts des conduits d'injection elles-mêmes réparties sur un même cercle : là encore, l'élasticité du matériau de la membrane maintient la fente fermée, et éventuellement de façon étanche. A la mise en pression du liquide par le déclenchement du moyen moteur, le liquide va écarter les bords de la fente et passer dans les conduits d'injection.

Avantageusement la membrane qui isole l'injection du principe actif, est au moins partiellement en appui sur la face amont (ou face intérieure) dudit injecteur.
Préférentiellement chaque conduit d'injection comporte à son extrémité amont une cavité d'entrée qui va faciliter le perçage de la membrane ou l'ouverture du préperçage de la membrane au moment de la pressurisation du liquide pour l'injection. Cette cavité d'entrée peut aussi être commune à plusieurs conduits, par exemple une rainure annulaire alimentant plusieurs conduits, ou même tous : cette disposition résout le problème du positionnement de la membrane par rapport à l'injection. La forme de cette cavité ou de cette rainure est optimisée pour permettre une bonne ouverture de la membrane et réaliser aussi une cavité en tête de conduit qui permet de piloter la distance de cohérence du jet de liquide sortant à l'autre extrémité du conduit.
Par exemple la cavité d'entrée, à l'amont du conduit d'injection, est délimitée par une surface de révolution dont la hauteur et le diamètre au niveau de la face amont de l'injecteur sont supérieurs à l'épaisseur de la membrane traversable, mais restent inférieurs à 2 ou 3 fois cette épaisseur.
Dans le cas d'une rainure circulaire pour alimenter les conduits d'injection, le profil optimisé de la rainure est un profil dont la hauteur et la largeur, mesurées selon un rayon de l'injecteur, sont supérieures à l'épaisseur de la membrane et restent inférieures à 2 ou 3 fois cette épaisseur.

Dans une réalisation différente de l'invention la face amont de l'injecteur ou fond intérieur de l'injecteur comporte des protubérances dirigées vers l'intérieur du réservoir. Les conduits d'injection débouchent dans le réservoir à travers ces protubérances. Ces protubérances peuvent être individuelles c'est-à-dire une protubérance pour chaque conduit ou communes à plusieurs ou à tous les conduits par exemple sous la forme d'une protubérance annulaire commune à tous les conduits disposés selon une circonférence centrée sur l'axe de réservoir. La membrane traversable qui isole le principe actif liquide de l'injecteur est initialement en appui sur les protubérances.
Quand le liquide est pressurisé pour réaliser l'injection la membrane se déplace et s'écrase contre les protubérances qui la percent ou ouvrent les préperçages de ladite membrane.
Comme précédemment la membrane peut comporter des zones de moindre épaisseur disposées en vis en vis de chaque protubérance, il faut alors une indexation relative précise des zones de moindre épaisseur et des protubérances : chacune de ces zones va être percée ou déchirée par la protubérance correspondante. La membrane peut comporter aussi une zone de moindre épaisseur unique sous forme d'une rainure : cette zone sera percée ou déchirée par les protubérances lors de la pressurisation du liquide.
La membrane traversable peut comporter des préperçages qui restent fermés tant que le liquide n'est pas pressurisé. Ces préperçages peuvent être individuels en ce sens que chaque préperçage est en vis à vis d'une protubérance ; il faut donc une indexation relative précise de la membrane vis à vis des protubérances. Ces préperçages peuvent être communs à toutes les protubérances par exemple sous forme d'une fente annulaire en vis à vis de toutes les protubérances ou même d'une protubérance annulaire.

Selon une première variante le moyen moteur agit directement sur l'obturateur amont pour réaliser l'injection.
Selon une deuxième variante le moyen moteur agit sur l'obturateur amont par l'intermédiaire d'un piston.
Préférentiellement le moyen moteur est un générateur pyrotechnique de gaz. Un tel dispositif est assez compact, puissant et surtout très fiable.

Avantageusement le réservoir et le corps de la seringue forment un ensemble unique.

Le réservoir est fretté dans le corps par un matériau intermédiaire lors du montage, sur le moyen moteur, de la seringue avec le réservoir.
Le corps de la seringue est transparent, ce qui permet la visualisation du réservoir de principe actif jusqu'à l'injection. Le corps comporte au moins une fenêtre de visualisation du contenu du réservoir, lorsque le matériau du corps de la seringue n'est pas transparent.
Avantageusement le piston agissant sur l'obturateur amont sert d'indicateur du fonctionnement de la seringue en apparaissant dans la partie transparente ou dans la fenêtre du corps de la seringue.

La présente invention résout les problèmes posés. Elle propose une seringue préremplie dont le réservoir est en un matériau compatible avec le principe actif et dont l'injecteur qui comporte plusieurs conduits d'injection, résiste à la pression élevée de fonctionnement, le problème de la compatibilité de cet élément étant résolu par l'interposition d'une membrane traversable compatible avec ledit principe actif.
La présente invention a aussi l'avantage de permettre de séparer, dans le dispositif, deux parties. Une partie qui sera dite partie pharmaceutique comprenant le corps, l'injecteur, sa membrane et le réservoir avec l'obturateur déplaçable à l'amont : ce sous-ensemble pourra être traité dans les conditions de l'industrie pharmaceutique notamment en ce qui concerne la stérilisation et l'asepsie. Ce sous-ensemble sera intégré au reste de la seringue, dont les éléments ont été assemblés par ailleurs, cet assemblage se faisant dans des conditions moins sévères que celle liées à l'industrie pharmaceutique.

Enfin cette configuration présente l'avantage d'éviter des fuites éventuelles de liquide par les conduits d'injection avant la réalisation de l'injection. En effet l'agitation du dispositif est fréquemment réalisée, voire préconisée, pour examiner la turbidité du liquide ou homogénéiser le mélange lorsque le liquide comporte des particules en suspension. Le fait que le principe actif est isolé, avant injection, des conduits réalise une protection ultime, vis à vis de ce risque de perte, pendant les manipulations qui précèdent l'application du dispositif sur la peau du sujet à traiter.

Ci-dessous l'invention est exposée en détail à l'aide de figures représentant différentes réalisations particulières de l'invention.
La figure 1 représente schématiquement et en coupe partielle une réalisation particulière d'une seringue sans aiguille selon l'invention.
La figure 2 représente en détail la partie de la seringue comportant le réservoir de principe actif, la membrane et l'injecteur.
La figure 3 illustre la déformation de la membrane en vis à vis de la cavité amont d'un conduit d'injection.
La figure 4 représente en détail la partie de la seringue comportant le réservoir, avec un injecteur comportant des protubérances.

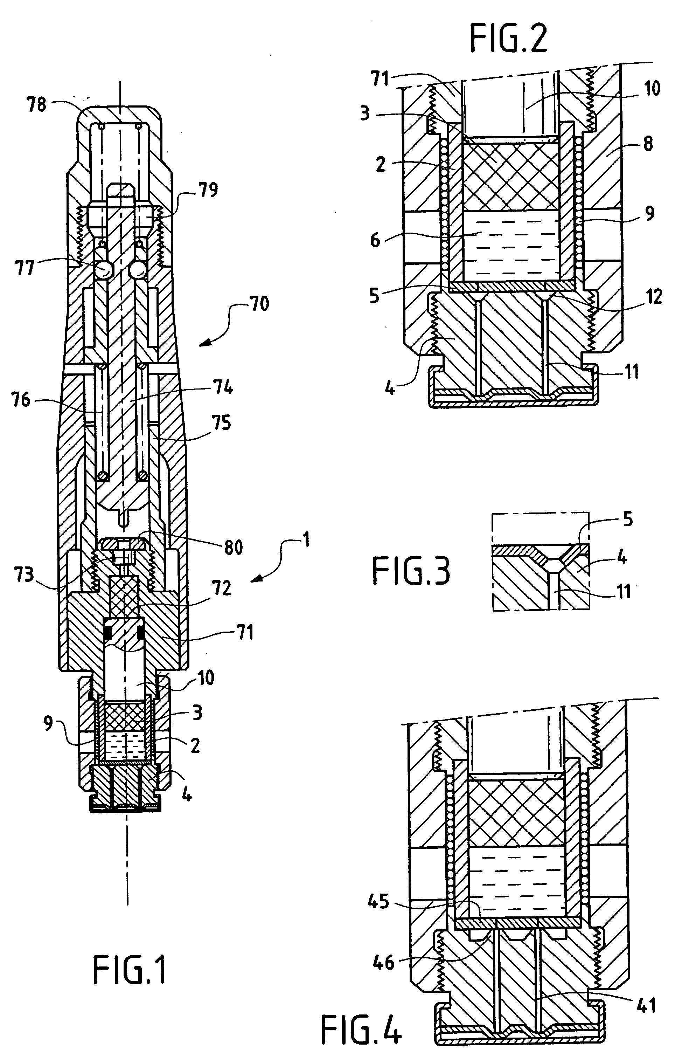

Selon les figures 1 et 2, la seringue est représentée verticale, le système d'injection ou injecteur 4 dirigé vers le bas : cette direction définit l'aval de la seringue, la direction opposée étant l'amont.
La seringue 1 comprend un corps 8 dans lequel est logé un réservoir cylindrique 2 contenant le principe actif liquide 6. A l'extrémité aval du corps 8 est monté un injecteur 4 comportant, dans cet exemple, quatre conduits d'injection tel, que le conduit 11. La face aval de l'injecteur est recouverte d'une protection extérieure pour assurer le maintien de l'asepsie de la seringue ; cette protection comprend une membrane, en élastomère, appliquée sur la face extérieure de l'injecteur par un opercule métallique fin serti autour de l'extrémité de la seringue. Cette protection sera retirée avant l'injection. A son extrémité amont le corps 8 est fixé à un moyen moteur 70 qui dans cet exemple est du type générateur pyrotechnique de gaz, il sera décrit par la suite.
Le réservoir 2 est, dans cet exemple, un tube d'un matériau transparent et compatible avec le principe actif liquide, ce matériau est par exemple du verre de type I ou II de la pharmacopée, mais on peut utiliser tout autre matériau transparent et compatible.
Le réservoir 2 est fermé à sa partie amont par un obturateur déplaçable 3. Cet obturateur 3 est du type bouchon-piston habituellement utilisé dans les seringues : c'est une pièce fabriquée par moulage d'un élastomère approprié pour faire piston et comportant plusieurs lèvres ou bourrelets latéraux pour réaliser l'étanchéité (ces éléments ne sont pas détaillés sur les figures). Cet obturateur peut recevoir, de façon connue, un traitement de surface pour faciliter son glissement dans le réservoir. Les matériaux habituellement utilisés pour réaliser les bouchons-pistons sont des élastomères connus dans la pharmacopée pour être comptible avec le principe actif : ce sont par exemple des chlorobutyl ou bromobutyl dont la dureté shore est réglée entre environ 45 et environ 70.
Le réservoir 2 est fermé à sa partie aval par une membrane traversable 5. Cette membrane traversable 5 est pincée entre le bord aval du réservoir 2 et la face amont essentiellement plane de l'injecteur 4 et réalise ainsi l'étanchéité du réservoir 2. La membrane traversable 5 est fabriquée avec un élastomère ou un polymère compatible avec le principe actif choisi par exemple dans le groupe précédemment cité. L'épaisseur de la membrane 5 est comprise entre environ 0,2mm et environ 1,5mm, préférentiellement cette épaisseur est comprise entre environ 0,2mm et environ 0,4mm.
L'injecteur 4 est, dans cet exemple, vissé sur le corps 8. L'injecteur comporte quatre conduits d'injection, tel que le conduit 11, répartis sur un cylindre dont l'axe coïncide avec celui du réservoir 2. Dans cet exemple les conduits sont représentés parallèles à l'axe du réservoir et de section sensiblement constante ; sans sortir du cadre de la présente invention ils peuvent avoir des configurations plus complexes pour des raisons et selon des modes de réalisation expliqués par ailleurs. Mais d'une façon générale pour ces conduits d'injection le rapport de la longueur au diamètre est supérieur à la dizaine, voire à quelques dizaines. Préférentiellement les conduits comportent, côté amont une cavité d'entrée 12. Chaque conduit peut avoir une cavité ou une cavité peut être commune à plusieurs conduits ou à tous les conduits, dans ce dernier cas il s'agira d'une rainure circulaire, compte tenu de ce qui a été décrit à propos de la disposition des conduits. Comme on le voit sur le détail de la figure 2 le rôle de cette cavité d'entrée est de permettre le déchirement ou l'ouverture du préperçage de la membrane 5 en vis à vis du conduit 11 lorsque le liquide est soumis à la pression d'injection. On peut remarquer alors que se crée sur le conduit, une alvéole, qui est favorable au pilotage de la distance de cohérence du jet comme nous l'avons expliqué par ailleurs.

Sur la face aval de l'injecteur 4, c'est-à-dire la face qui sera en appui sur la peau du sujet à traiter, les conduits 11 débouchent par des protubérances qui éventuellement se rejoignent pour former une collerette circulaire. Cette disposition est favorable pour réaliser un appui plus uniforme sur la peau et permet de tolérer un petit défaut de perpendicularité de la seringue vis à vis de la peau sans nuire à la qualité de l'injection.

Dans cet exemple le moyen moteur 70 agit sur l'obturateur 3 par l'intermédiaire d'un piston 10, représenté partiellement en coupe. Ce piston est métallique ou en matière plastique dure telle que le Téflon®, il comporte des moyens d'étanchéité, simplement représenté ici par un joint torique. Ce piston empêche, par ses moyens d'étanchéité, les gaz de combustion du générateur pyrotechnique de venir au contact de l'obturateur 3. Ce piston sert aussi de témoin de fonctionnement lorsqu'il apparaît dans la fenêtre du corps 8. Ce rôle de témoin visuel peut aussi être assuré par l'obturateur déplaçable lui-même.

Enfin, les figures 1 et 2 présentent un cas particulier de montage du réservoir 2 dans le corps 8. Le réservoir 2, centré dans l'injecteur 4 et placé dans le corps 8, est enveloppé d'un matériau intermédiaire 9, qui lorsque l'ensemble sera monté sur le corps 71 du moteur 70 sera comprimé par la lèvre du corps 71 : cette compression du matériau intermédiaire 9 réalise à ce moment le frettage du réservoir 2 et lui permet de résister aux pressions élevées d'injection lors du fonctionnement du moteur.

Nous allons décrire les principaux éléments, du générateur pyrotechnique. Il comprend le corps 71, un chargement pyrotechnique 72 dont la combustion est initiée par une amorce 73 impactée par un percuteur 74. L'amorce 73 est recouverte par dans un porte-amorce 80. En position initiale le percuteur 74 est retenu, dans le guide-percuteur 75 solidaire par vissage du corps 71, par au moins une bille, telle que la bille 77, partiellement engagée dans une gorge du percuteur. Le dispositif de percussion comprend un poussoir 78 avec une gorge 79 et un ressort intérieur 76. Le poussoir 78 coulisse sur l'extérieur du guide-percuteur 75 et il est retenu par des ergots se déplaçant dans des rainures latérales. Ce poussoir 78 est ici l'organe de déclenchement.
Bien entendu pour initier la combustion du chargement pyrotechnique 72, sans sortir du cadre de l'invention, on peut utiliser des dispositifs d'initiation autres que le dispositif à percuteur ici décrit. Sans entrer dans les détails et sans vouloir être exhaustif, nous citerons comme exemples des dispositifs d'initiation à pile électrique ou des dispositifs d'initiation piézo-électrique.
Eventuellement le générateur de gaz pyrotechnique peut être remplacé par un générateur de gaz constitué par un réservoir de gaz comprimé fermé par une vanne à ouverture rapide. L'organe de déclenchement va ouvrir ladite vanne, les gaz comprimés du réservoir vont se détendre et agir sur le moyen de poussée.

Pour l'utilisation après avoir enlevé le bouchon d'asepsie et disposé la seringue sur la peau du sujet à traiter, l'opérateur appui, avec son pouce, sur le poussoir 78 qui s'enfonce en comprimant le ressort 76. Le poussoir se déplace jusqu'à ce que la gorge 79 arrive à la hauteur de la gorge contenant les billes, telle que la bille 77, billes qui se dégagent dans la gorge 79 et libèrent le percuteur qui va impacter violemment l'amorce 73, dont l'initiation enflamme le chargement pyrotechnique 72. Le percuteur en appui sur le porte-amorce 80 le maintient dans son logement et assure l'étanchéité : les gaz de combustion ne remontant pas vers le poussoir.

Le figure 3 représente en détail l'ouverture d'une membrane au droit d'un préperçage. Dans la membrane sont percés des trous circulaires dont le diamètre est compris entre environ 100 µm et environ 300 µm ; du fait de l'élasticité de l'élastomère de la membrane les préperçages restent fermés et relativement étanches. En tête du conduit 11, en vis à vis d'un préperçage de la membrane se trouve une cavité d'entrée 12 de forme essentiellement conique dont la hauteur est égale à environ deux fois le diamètre de la base. Sous l'effet de la pression transmise au liquide, lors de la mise en fonctionnement du moyen moteur, le bord du préperçage de la membrane se déforme et vient s'appliquer sur la paroi de la cavité conique en tête de conduit. A l'entrée du conduit se forme alors une alvéole qui produit des perturbations et des turbulences dans l'écoulement ; ces phénomènes contribuent au pilotage de la distance de cohérence du jet.

La figure 4 représente une vue d'un détail d'une autre réalisation de l'invention. Dans cet exemple l'injecteur de la seringue comporte des protubérances 46 du côté amont de chaque conduit d'injection 41 ; dans cet exemple la protubérance a une forme très simple celle d'un tronc de cône. La membrane traversable 45 qui comporte des zones prépercées en vis à vis des conduits d'injection est en appui sur ces protubérances. Le pourtour extérieur de la membrane 45 est pincée entre le tube du réservoir et un rebord périphérique de l'injecteur. La membrane est calée angulairement vis à vis de l'injection par un système de pion ou d'ergot (non représenté sur ce schéma) de façon que les préperçages de la membrane soient en vis à vis des conduits d'injection. Sous l'effet de la pressurisation du liquide par le moyen moteur, la membrane va s'écraser sur l'injecteur, les zones prépercées sont ouvertes par les protubérances coniques ; les bords des zones prépercées s'écartent pour laisser passer le liquide.

## Revendications

1. Seringue sans aiguille (1) pour l'injection d'un principe actif liquide (6), ladite seringue comprenant un réservoir cylindrique et tubulaire (2) apté à contenir ledit principe actif, le réservoir étant fermé à sa partie amont par un obturateur déplaçable (3) par un moyen moteur (70), et fermé à sa partie aval par une membrane traversable (5) compatible avec le principe actif et recouvrant un injecteur (4) comprenant au moins un conduit d'injection et ce réservoir étant solidarisé au corps (8) de la seringue, ladite membrane (5) comportant des préperçages en regard de chaque conduit d'injection (11), chaque préperçage restant fermé et étanche tant que le liquide n'est pas mis en pression, cette membrane (5) étant pincée entre le bord aval du réservoir (2) et la face amont essentiellement plane de l'injecteur (4), ladite seringue étant **caractérisée en ce que** la membrane (5) recouvre au moins deux conduits d'injection dont les extrémités amont sont situées sur un même cercle centré sur l'axe du réservoir cylindrique.

2. Seringue sans aiguille selon la revendication 1 **caractérisée en ce que** la membrane (5) comporte une fente circulaire centrée sur l'axe du réservoir, cette fente est en regard des extrémités amont des conduits d'injection et elle reste fermée et étanche tant que le liquide n'est pas mis en pression.

3. Seringue sans aiguille selon l'une des revendications 1 ou 2 **caractérisée en ce que** la cavité d'entrée (12) est délimitée par une surface de révolution dont la hauteur et le diamètre au niveau de la face amont sont supérieurs à l'épaisseur de la membrane et inférieurs à 3 fois cette épaisseur.

4. Seringue sans aiguille selon la revendication 3 **caractérisée en ce que** les cavités d'entrées se rejoignent et forment une rainure circulaire sur la face amont de l'injecteur (4).

5. Seringue sans aiguille selon la revendication 4 **caractérisée en ce que** la rainure circulaire a une hauteur et une largeur supérieures à l'épaisseur de la membrane et inférieures à 2 fois cette épaisseur.

6. Seringue sans aiguille selon l'une des revendications 1 ou 2 **caractérisée en ce que** la membrane (45) est en appui sur des protubérances (46) situées à l'extrémité amont de chaque conduit (41).

7. Seringue sans aiguille selon l'une des revendications 1 à 6 **caractérisée en ce que** le moyen moteur (70) agit directement sur l'obturateur amont (3).

8. Seringue sans aiguille selon l'une des revendications 1 à 6 **caractérisée en ce que** le moyen moteur (70) agit sur l'obturateur amont par l'intermédiaire d'un piston (10).

9. Seringue sans aiguille selon l'une des revendications précédentes **caractérisée en ce que** le moyen moteur (70) est un générateur pyrotechnique de gaz.

10. Seringue sans aiguille selon l'une des revendications précédentes **caractérisée en ce que** le réservoir (2) et le corps (8) forment un ensemble unique.

11. Seringue sans aiguille selon l'une des revendications 1 à 10 **caractérisée en ce que** le réservoir (2) est fretté dans le corps (8) par un matériau intermédiaire (9) lors du montage sur le moyen moteur (70).

12. Seringue sans aiguille selon l'une des revendications 1 à 10 **caractérisée en ce que** le corps (8) est transparent.

13. Seringue sans aiguille selon l'une des revendications 1 à 11 **caractérisée en ce que** le corps (8) comporte au moins une fenêtre de visualisation du contenu du réservoir.

## Patentansprüche

1. Nadellose Spritze (1) zum Spritzen eines flüssigen Wirkstoffs (6), wobei die Spritze einen zylindrischen und rohrförmigen Behälter (2) aufweist, der den Wirkstoff enthalten kann, wobei der Behälter in seinem vorderen Bereich von einer mittels eines Antriebsmittels (70) verschiebbaren Verschlussvorrichtung (3) und in seinem hinteren Bereich von einer durchquerbaren Membran (5) verschlossen wird, die mit dem Wirkstoff kompatibel ist und einen Injektor (4) bedeckt, der mindestens einen Spritzkanal aufweist, und wobei dieser Behälter fest mit dem Körper (8) der Spritze verbunden ist, wobei die Membran (5) Vorbohrungen vor jedem Spritzkanal (11) aufweist, wobei jede Vorbohrung so lange geschlossen und dicht bleibt, wie die Flüssigkeit nicht unter Druck gesetzt wird, wobei diese Membran (5) zwischen den hinteren Rand des Behälters (2) und die im Wesentlichen ebene Vorderseite des Injektors (4) geklemmt ist, wobei die Spritze **dadurch gekennzeichnet ist, dass** die Membran (5) mindestens zwei Spritzkanäle bedeckt, deren vordere Enden sich in einem gleichen Kreis befinden, der auf die Achse des zylindrischen Behälters zentriert ist.

2. Nadellose Spritze nach Anspruch 1, **dadurch gekennzeichnet, dass** die Membran (5) einen auf die Achse des Behälters zentrierten kreisförmigen Schlitz aufweist, wobei dieser Schlitz gegenüber den vorderen Enden der Spritzkanäle liegt und so lange geschlossen und dicht bleibt, wie die Flüssigkeit nicht unter Druck gesetzt wird.

3. Nadellose Spritze nach einem der Ansprüche 1 oder 2, **dadurch gekennzeichnet, dass** der Eingangshohlraum (12) von einer drehsymmetrischen Fläche begrenzt wird, deren Höhe und Durchmesser in Höhe der vorderen Fläche größer sind als die Dicke der Membran und geringer als das Dreifache dieser Dicke.

4. Nadellose Spritze nach Anspruch 3, **dadurch gekennzeichnet, dass** die Eingangshohlräume aneinander anschließen und auf der vorderen Fläche des Injektors (4) eine kreisförmige Rille bilden.

5. Nadellose Spritze nach Anspruch 4, **dadurch gekennzeichnet, dass** die kreisförmige Rille eine Höhe und eine Breite hat, die größer sind als die Dicke der Membran und kleiner als deren doppelte Dicke.

6. Nadellose Spritze nach einem der Ansprüche 1 oder 2, **dadurch gekennzeichnet, dass** die Membran (45) auf Ausstülpungen (46) aufliegt, die sich am vorderen Ende jedes Kanals (41) befinden.

7. Nadellose Spritze nach einem der Ansprüche 1 bis 6, **dadurch gekennzeichnet, dass** das Antriebsmittel (70) direkt auf die vordere Verschlussvorrichtung (3) einwirkt.

8. Nadellose Spritze nach einem der Ansprüche 1 bis 6, **dadurch gekennzeichnet, dass** das Antriebsmittel (70) über einen Kolben (10) auf die vordere Verschlussvorrichtung einwirkt.

9. Nadellose Spritze nach einem der vorhergehenden Ansprüche, **dadurch gekennzeichnet, dass** das Antriebsmittel (70) ein pyrotechnischer Gasgenerator ist.

10. Nadellose Spritze nach einem der vorhergehenden Ansprüche, **dadurch gekennzeichnet, dass** der Behälter (2) und der Körper (8) eine einzige Einheit bilden.

11. Nadellose Spritze nach einem der Ansprüche 1 bis 10, **dadurch gekennzeichnet, dass** der Behälter (2) bei der Montage auf das Antriebsmittel (70) mittels eines Zwischenmaterials (9) in den Körper (8) eingeschrumpft wird.

12. Nadellose Spritze nach einem der Ansprüche 1 bis 10, **dadurch gekennzeichnet, dass** der Körper (8) transparent ist.

13. Nadellose Spritze nach einem der Ansprüche 1 bis 11, **dadurch gekennzeichnet, dass** der Körper (8) mindestens ein Sichtfenster für den Inhalt des Behälters aufweist.

## Claims

1. A needleless syringe (1) for injection of a liquid active ingredient (6), said syringe comprising a cylindrical and tubular reservoir (2) capable of containing said active ingredient, the reservoir being closed at its upstream part by an obturator (3), which is capable of being displaced by a driving means (70), and closed at its downstream part by a traversable membrane (5) which is compatible with the active ingredient and which covers an injector (4) comprising at least one injection duct, said reservoir being positively connected to the body (8) of the syringe, said membrane (5) including prepared bores opposite each injection duct (11), each prepared bore remaining closed and impervious as long as the liquid is not pressurised, said membrane (5) being gripped between the downstream edge of the reservoir (2) and the essentially plane upstream face of the injector (4), said syringe being **characterised in that** the membrane (5) covers at least two injection ducts, the upstream ends of which are situated on the same circle centred on the axis of the cylindrical reservoir.

2. Needleless syringe according to Claim 1, **characterised in that** the membrane (5) includes a circular aperture centred on the axis of the reservoir, said aperture being opposite the upstream ends of the injection ducts and remaining closed and impervious as long as the liquid is not pressurised.

3. Needleless syringe according to one of Claims 1 or 2, **characterised in that** the inlet cavity (12) is delimited by a surface of revolution, the height of which and the diameter of which at the level of the upstream face are greater than the thickness of the membrane and less than three times this thickness.

4. Needleless syringe according to Claim 3, **characterised in that** the inlet cavities join together and form a circular groove on the upstream face of the injector (4).

5. Needleless syringe according to Claim 4, **characterised in that** the circular groove has a height and a width which are greater than the thickness of the membrane and less than twice this thickness.

6. Needleless syringe according to one of Claims 1 or 2, **characterised in that** the membrane (45) is supported on protuberances (46) situated at the upstream end of each duct (41).

7. Needleless syringe according to one of Claims 1 to 6, **characterised in that** the driving means (70) acts directly on the upstream obturator (3).

8. Needleless syringe according to one of Claims 1 to 6, **characterised in that** the driving means (70) acts on the upstream obturator via a plunger (10).

9. Needleless syringe according to one of the preceding claims, **characterised in that** the driving means (70) is a pyrotechnic gas generator.

10. Needleless syringe according to one of the preceding claims, **characterised in that** the reservoir (2) and the body (8) form a single assembly.

11. Needleless syringe according to one of Claims 1 to 10, **characterised in that** the reservoir (2) is hooped within the body (8) by means of an intermediate material (9) in the course of mounting on the driving means (70).

12. Needleless syringe according to one of Claims 1 to 10, **characterised in that** the body (8) is transparent.

13. Needleless syringe according to one of Claims 1 to 11, **characterised in that** the body (8) includes at least one window for viewing the contents of the reservoir.
